# EUROPEAN PATENT SPECIFICATION

(11) **EP 0 682 649 B1**
(45) Date of publication and mention of the grant of the patent: **02.09.1998**
(21) Application number: 94909511.1
(22) Date of filing: 25.01.1994
(51) Int. Cl.: C07B 59/00, A61K 31/08, C07C 43/12

(54) **DEUTERATED SEVOFLURANE AS AN INHALATIONAL ANESTHETIC**
DEUTERIERTES SEVOFLURAN ALS INHALATIONSBETÄUBUNGSMITTEL
SEVOFLURANE DEUTERIE UTILISE COMME ANESTHESIANT INHALE

(30) Priority: 28.01.1993 US 10264; 14.06.1993 US 76582; 12.08.1993 US 79165
(43) Date of publication of application: 22.11.1995
(73) Proprietor: UNIVERSITY OF IOWA RESEARCH FOUNDATION, Iowa City, IA 52242 (US)
(72) Inventor: TINKER, John, H., Iowa City, IA 52245 (US); BAKER, Max, T., Iowa City, IA 52246 (US)
(74) Representative: Lawrence, Malcolm Graham
(86) International application number: US9401050
(87) International publication number: WO9417016

(56) References cited:
- EP-A- 0 013 921
- US-A- 3 683 092
- US-A- 4 153 636
- US-A- 4 154 971

## Description

The invention relates to fluorodideuteromethyl1,1,1,3,3,3,-hexafluoro-2-propyl ether and its hexafluoro-2-deutero-2-propyl ether analog, and to the use thereof in anaesthesia.

Halogenated isopropyl derivatives of ether have demonstrated promise for use in the medical field due to their anaesthesia inducing properties. Of these, the most successful to date has been with fluorinated isopropyl ethers such as sevoflurane (fluoromethyl1,1,1,3,3,3, -hexafluoro-2-propyl ether). Sevoflurane has demonstrated rapid induction and recovery from anaesthesia when administered by inhalation, making it attractive for use as an anaesthetic. Further, sevoflurane is a volatile liquid, non-flammable in air at ambient temperatures and has a lower flammability limit in oxygen of about 11.8 volume percent, making it safe to use as well. United States Patent 3,683,092 to Regan et al. discloses use of sevoflurane as an anaesthetic.

While exhibiting many beneficial anaesthetic properties such as the ability to rapidly change depth of anaesthesia, use of sevoflurane as a general inhalational anaesthetic has been hampered by its potential nephrotoxicity when metabolised at sufficiently high levels. It is, however, commonly used in Japan.

Attempts to find other halogenated isopropyl derivatives with beneficial anaesthetic properties have led scientists to substitute sevoflurane with other similar moieties. These attempts have not been successful in that several related compounds either do not possess any anesthetic properties, produce only small anesthetic properties, or are toxic. For example, U.S. Patent 3,683,092 discloses that the compound CH₃OCF(CF₃)₂ was found to be non-anesthetic up to 8% by volume in oxygen meaning that it would burn at its anesthetic concentration since its lower flammability limit is about 7-8%. Another isomer, trifluoromethyl -2, 2,3,3-tetrafluoropropyl ether of Aldrich and Shepard, J. Or. Chem., Volume 29, pages 11-15 (1964) has been shown to cause violent convulsions and death in mice at concentrations as low as .5%. Yet another isomer, CHF₂OCH₂CF₂CF₃ is non-anesthetic up to its lethal concentration and produces convulsions in mice. Still another comparison, it has been found that the Isomeric (CHF₂)₂CF-O-CHF₂ is a weak anesthetic in which deep anesthesia is not obtained and abnormal electro-encephalographic and convulsant activity is observed. Thus it can be seen that there has been little success to date, and a need exists for an anesthetic for use in mammals which will possess the advantageous characteristics of sevoflurane while minimizing the concomitant fluoride ion release.

Another problem that exists with sevoflurane, relates to degradation products when used in a typical anesthetic circuit involving CO₂ absorbants, see Morio, et al, "Reaction of Sevoflurane and its Degradation Products with Soda Lime: Toxicity of the By-Products," Anesthesiology 77:1155-1164, 1992 and Frink et al, "Quantification of the Degradation Products of Sevoflurane in two CO₂ Absorbants During Low-flow Anesthesia in Surgical Patients," Anesthesiology 77:1064-1069, 1992. Although these degradation products eventually include perhaps up to five different compounds, the initial reaction leads to the most prevalent and potentially harmful compound which is known in the art as "Compound A". It is an unsaturated compound of the formula fluoromethyl-2, 2 difluoro-1-trifluoromethyl vinyl ether represented as follows:

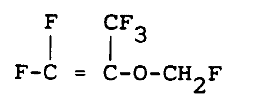

By way or background, a typical anesthetic circuit includes, of course, the anesthetic machine which administers an inhalational anesthetic to the patient, the patient's respiratory system for inhaling of a mixture of oxygen and anesthetic and exhaling anesthetic mixture now having a high carbon dioxide content, a scrubber system to remove carbon dioxide from the exhaled gas mixture then, of course, a recycle system using one-way valves back to the anesthetic circuit. In this semi-closed loop process, the typical carbon dioxide scrubber or absorbant is a canister of a mixture known generally as soda lime. There are perhaps as many as five commercial manufacturers of soda lime for use in anesthesiology. Generally, those mixtures include a mixture of sodium, calcium and potassium hydroxide. Some examples of soda lime commercially available and commonly used in surgery for CO₂ absorption or scrubbing are Sodasorb^{TM} by W.R. Grace of Lexington, Massachusetts, Barolyme of Chemtron Medical Division, Allied Health Care Products of St. Louis, Missouri, and Wakolime- (Wako Pure Chemical, Osaka, Japan). Soda lime comprises about 5% sodium hydroxide and about 95% calcium hydroxide. Depending upon the manufacturer, other components present in carbon dioxide scrubber canisters include potassium hydroxide, barium hydroxide, sodium silicate, and of course, water. All are very strong bases.

All of these products operate in generally the same way, that is the sodium hydroxide primarily reacts with carbon dioxide, which produces bicarbonate which in turn reacts with the calcium hydroxide to provide insoluble precipitated carbonates. The reaction is exothermic, and although the canister starts at ambient conditions, by the time the surgery is underway it warms and often reaches a stable temperature within the range of 45°C to 50°C. The exact steady state temperature reached depends upon the CO₂ volume produced by the patient and the total flow of all gases in the air.

As reported in the earlier referenced articles of Morio et al and Frink et al, at elevated temperatures in soda lime, one commonly observes degraded products of sevoflurane which are potentially toxic. In particular there are problems with two different parts of the sevoflurane molecule. The fluoromethoxy carbon portion of the molecule degrades during metabolism in the patient's liver to release fluoride ion which can potentially damage the kidney. The hexafluoroisopropyl portion of the compound, in the presence of soda lime under normal operating conditions, will degrade to provide "Compound A," a vinyl unsaturate, which has been demonstrated in the literature to be toxic when inhaled at ranges of from 100 ppm to 1000 ppm during inhalational experiments with rats. Obviously, therefore, although sevoflurane has significant opportunities as an inhalational anesthetic, the organ toxicity issues associated with the use of sevoflurane must first be solved.

We have discovered that deuterated sevoflurane is metabolised and subsequently defluorinated at a much slower rate than sevoflurane itself, thereby reducing fluoride ion release while still maintaining all of the anaesthetic properties of sevoflurane. This solves a very important problem with this molecule, namely, liver metabolism to release fluoride ion. The present invention represents an improvement on sevoflurane by use of deuterated sevoflurane.

The present invention aims to provide a deuterated sevoflurane compound with the beneficial properties of sevoflurane for use as an inhalational anaesthetic which will reduce metabolic inorganic fluoride release, as well as a method for inducing anaesthesia in patients involving inhalation of such compound, anaesthesia being produced in a patient while being slowly metabolised.

The present invention also aims to provide a method of synthesis of fluorodideuteromethyl-1,1,1,3,3,3,-hexafluoro-2-propyl ether.

This invention relates to a method of synthesis of deuterated sevoflurane (ie D₂ sevoflurane and D₃ sevoflurane - the two compounds defined by the formula set forth below) and use of the same for anaesthetising animals. D₂ sevoflurane is metabolised and subsequently defluorinated at a much slower rate thereby reducing fluoride ion release, while maintaining all of the anaesthetic properties of sevoflurane.

According to the invention, there is provided a deuterated sevoflurane having the following formula:- 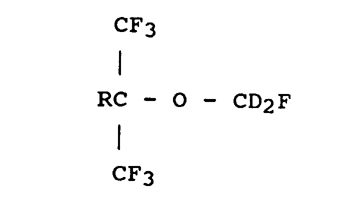 wherein R is hydrogen or deuterium.

A method of inducing anaesthesia in animals is disclosed in which D₂ or D₃ sevoflurane is administered by inhalation to the animals. Further, fluorodideuteromethyl-1,1,3,3,3,-hexafluoro-2-propyl ether is synthesised by reacting dimethyl-D₆-sulfate with 6 1,1,1,3,3,3,-hexafluoro-isopropanol, which is then reacted with BrF₃. Excess BrF₃ is then destroyed leaving fluorodideuteromethyl-1,1,1,3,3,3,-hexafluoro-2-propyl ether.

The invention will now be described by way of example, reference being made to the accompanying drawings, in which:-
Figure 1 is a graph depicting concentration dependent defluorination of deuterated sevoflurane, sevoflurane, and enflurane in hepatic microsomes from Isoniazid treated rats;
Figure 2 is a graph depicting concentration dependent defluorination of deuterated sevoflurane, sevoflurane, and enflurane in hepatic microsomes from phenobarbital treated rats; and
Figure 3 is a bar graph depicting Plasma fluoride levels in rats anesthetized for 30 minutes with deuterated sevoflurane, sevoflurane, and enflurane.

### DETAILED DESCRIPTION OF THE INVENTION

Sevoflurane, or fluoromethyl-1,1,1,3,3,3-hexafluoro-2-propyl ether has the following formula:

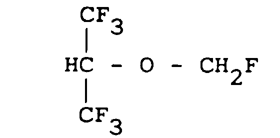

Sevoflurane, upon administration into the body, is metabolized in the liver by cytochrome P450, liberating fluoride and hexafluoroisopropanol. Inorganic fluoride at sufficiently high levels will produce renal dysfunction including polyuric renal failure, which can be fatal.

In addition, as illustrated in the earlier referenced articles of Morio et al and Frink et al, double bonded breakdown products starting with "Compound A", which are inhaled after formation in sodalime, can irreversibly bind to tissue macromolecules resulting in major organ toxicity. Although the toxicity of these degradation by-products has been mentioned in both the Morio and the Frink articles, nothing is suggested as to how or why those degradation products occur.

In vitro tests have demonstrated that deuterated sevoflurane, particularly with deuterium substitutions at the monofluoro substituted methyl group may be used in animals and as an inhalational anaesthetic. One such deuterium substituted derivative found to be especially useful is fluoro-dideutero-methyl-1,1,1,3,3,3,-hexafluoro-2-propyl ether of which the following is a formula:-

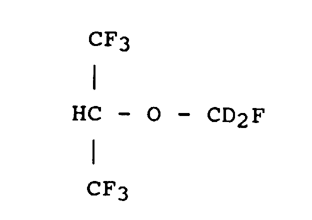

The compound retains all of the beneficial anaesthetic qualities of sevoflurane as discussed earlier while at the same time markedly decreasing the exposure to fluoride. This result is surprising due to the fact that isomers of halogenated isopropyl ethers are largely unpredictable with respect to their anesthetic qualities. Further it has been demonstrated that deuterium substitution of ethers used as anesthetic compounds also quite unpredictable in their altered kinetics of metabolism.

U.S. Patent 4,154,971 to Larsen et al. discloses monodeuterated analogs of 1,1 difluoro 2,2-dihaloethyl difluoromethyl ethers. These are analogs of the anesthetics euflurane and isoflurane, not sevoflurane. Accordingly it was discovered that 1,1,2-trifluoro-2-chloro-2-deuteroethyl-difluoromethyl ether; 1,1-difluoro-2-deutero-2,2-dichloroethyl difluoromethyl ether; and 1,1,2-trifluroro-2-bromo-2-deuteroethyl difluoromethyl ether all exhibited the characteristic of slower metabolism and thus slower defluorination. However, 1,1-difluoro-2,2-dichloro-2-deuteroethyl methyl ether exhibited properties of being more readily metabolized into inorganic fluoride than the undeuterated compound. This unpredictability of deuteration on fluoride ion release has similarly been encountered in other patented systems.

U.S. Patent 4,153,636 similarly discloses deuterated analogs of the anesthetic methoxyflurane wherein 2,2-dichloro-1,1-difluoro-1-methoxy-D₃-ethane and 2,2-dichloro-1,1 difluoro-1-methoxy-D₃-ethane were found to have decreased metabolism and again 1,1 difluoro-2-2-dichloro-2-deuteroethyl methyl ether was found to increase inorganic fluoride release.

The unpredictability of deuteration of anesthetics can also be demonstrated by the deuteration of halothane. Deuteration of halothane inhibits its metabolism to trifluoroacetic acid, but not its metabolism to release fluoride (Sipes IG, Gandolfi AJ, Pohl LR, Krishna G, Brown Jr BR: "Comparison of the Biotransformation and Hepatotoxicity of Halothane and Deuterated Halothane." J. Pharmacol. Exp. Ther. 214: 716-720, 1980).

Thus it can be seen that the placement of deuterium atoms in the molecule is critical and highly species specific. D₂ sevoflurane with deuterium atoms at the monofluoro methyl group produces an unexpected unpredictable result of decreased rate of metabolism and decreased fluoride ion release while maintaining all the beneficial anesthetic properties of the compound.

According to the invention of our parent application, substitution of the hydrogens at the methyl group of sevoflurane with deuterium(D), a heavy isotope of hydrogen, alters the kinetics of metabolism of the compound. The compound retains its anesthetic qualities while being metabolized much more slowly thereby reducing production of inorganic fluoride. Substitution of the hydrogens at the methyl group of sevoflurane eliminates the concentration-dependent peak of fluoride release which occurs upon sevoflurane and enflurane metabolism in microsomes from isoniazid treated rats. Liver microsomes from isoniazid treated rats contain the same cytochrome P450 isozyme, P4502E1 which is present in humans and is inducible by ethanol, isoniazid and other compounds in humans.

D₂-sevoflurane may be synthesized by modification of a method for synthesis of sevoflurane described in United States Patent 3,683,092, the disclosure of which is incorporated herein by reference. The method is similar except, Dimethyl-D₆-sulfate, instead of dimethyl sulfate is reacted with 1,1,1,3,3,3-hexafluoroisopropanol to form trideuteromethyl-1,1,1,3,3,3-hexafluoro-2-propyl ether. The resulting trideuteromethyl-1,1,1,3,3,3-hexafluoro-2-propyl ether is then subsequently monofluorinated by reaction with bromine trifluoride.

D₃-sevoflurane is synthesized by reacting D₂-sevoflurane with NaOD in D₂O. The D₂ sevoflurane is deuterated in the 2 propyl position to form fluoro-dideuteromethyl-1,1,1,3,3,3-hexafluoro-2-deutero-2-propyl ether. All reactions are run with equimolar quantities of reactants preferred, although excesses of one or more of the variants may be used. No critical limits as to temperature or pressure exist, traditionally ambient conditions are used.

The end product, D₂-sevoflurane or D₃-sevoflurane may then be administered by the inhalation route to warm blooded, air breathing animals, in an effective anesthetizing amount. Generally the compound is administered in an amount of from about 1 percent to about 5 percent by volume in admixture with from 99 percent to about 95 percent by volume of oxygen or a gaseous mixture containing oxygen and/or other anesthetics (e.g. nitrous oxide).

The following examples are offered to further illustrate but not limit the process of this invention.

### Example 1

To prepare trideuteromethyl-1,1,1,3,3,3-hexafluoro-2-propyl ether, hexafluoroisoproponal (53.3g) was added to 127 ml of 10% aqueous sodium hydroxide in a Pyrex flask. Dimethyl-D₆-sulfate (40g) was added proportion wise during a thirty-minute period at 5 °C while stirring. The reaction mixture was stirred for two hours at room temperature. Distillation of reaction mixture yielded 45g of trideuteromethyl-1,1,1,3,3,3-hexafluoro-2-propyl ether.

D₂-sevoflurane was obtained by placing 8 ml of dried trideuteromethyl-1,1,1,3,3,3-hexafluoro-2-propyl ether in a Pyrex flask. 3 ml of BrF₃ were slowly added over a two hour period while stirring. An exothermic reaction occurred, monofluorinating the ether compound. Following the reaction, water was cautiously added to destroy excess BrF₃ in the reaction mixture. The reaction mixture was successively washed with dilute sodium sulfate and water. Finally the washed mixture was dried over anhydrous sodium sulfate and yielded 3.1 ml D₂-sevoflurane.

### Example 2

The formation of D₂-sevoflurane and determination of its purity were evaluated by two methods of gas chromatography, and by GC-mass spectrometry using the electron impact (EI) and chemical ionization (CI) modes.

The synthesized product exhibited a retention time identical to that of sevoflurane on gas chromatography. D₂-Sevoflurane chromatography on a carbowax column and using flame ionization detection, showed that the D₂-sevoflurane was 99.86% pure. The contaminant at 7.0 minutes and constituting 0.032% of the sample was identified as hexafluoroisopropanol. Chromatography of the synthesized D₂-sevoflurane sample on 10% CO-880 15% LB-550X indicates a purity of 99.9%. This column resolves methyl hexafluoroisopropyl ether or trideuteromethyl hexafluoropropyl ether (retention time of 2.2 minutes) from sevoflurane or D₂-sevoflurane (3.3 minutes) and showed that the sample contained no trideuteromethyl-1,1,1,3,3,3-hexafluoro-2-propyl ether (or methyl hexafluoroisopropyl ether).

### Example 3

Mass spectral analysis of the synthesized D₂-sevoflurane was performed on a Nermag R10-10C mass spectrometer in the electron impact and chemical ionization modes. The mass spectrometer was equipped with a DB Wax 30 m x 0.2 mm x 0.5 µm capillary column for sample introduction.

The electron impact mass spectra of sevoflurane and D₂-sevoflurane were observed. The parent ion of either compound was not observed; however, the M-F and M-CF3 fragments occurred. Sevoflurane analysis yielded a M-F fragment with m/z of 181, whereas D₂-sevoflurane produced a fragment of m/z 183 - two atomic mass units greater. Also, sevoflurane generated a m/z fragment of 131 (M-CF3), whereas D₂-sevoflurane showed the corresponding fragment at m/z 133 - also two atomic mass units greater. The greater mass of 2 for these fragments confirms that the deuterated compound is fluorodideuteromethyl-1,1,1,3,3,3-hexafluoro-2-propyl ether, and the spectra showed that in the D₂-sevoflurane sample no sevoflurane was detectable.

Mass spectra of sevoflurane and D₂-sevoflurane in the chemical ionization mode showed the parent ion m/z (M+1) of 201 for sevoflurane and 203 for D₂-sevoflurane. The parent ion of D₂-sevoflurane was 2 atomic mass units greater than that of sevoflurane again confirming D₂-sevoflurane.

### Example 4

### METABOLISM OF D₂-SEVOFLURANE

The metabolism of D₂-sevoflurane is expected to liberate one fluoride ion for each molecule metabolized by cytochrome P450 since the metabolism of sevoflurane liberates fluoride and hexafluoroisopropanol. To determine the metabolism of D₂-sevoflurane relative to sevoflurane and enflurane, these anesthetics were incubated with hepatic microsomes from untreated male Sprague-Dawley rats (200-230g), or rats treated with isoniazid (80mg/kg, i.p. for 5 days), or phenobarbital (0.2% in the drinking water for 4 days). Isoniazid induces the cytochrome P450 isozyme P450 2E1 which is thought to metabolize the volatile anesthetics, and phenobarbital induces several forms also shown to play a role in anesthetic metabolism in the rat.

Each incubation vial (6 ml plastic vial) contained 3 ml of 5 mg/ml microsomal protein in a 0.1 M sodium phosphate buffer, pH 7.4. An NADPH generating system was added to cytochrome P450 activity, and the NADPH generating system was omitted from control incubations. Anesthetic was added in the quantities indicated and microsomes were incubated for 15 minutes at 37°C. Reactions were stopped by placing the vials on ice. Fluoride was assayed in the microsomal mixtures using fluoride ion-specific electrodes (Fisher Scientific) and a 720A Orion pH/ISE meter. Following incubation microsomes were mixed with an equal volume of TISABII buffer for fluoride analysis. Fluoride in each sample was determined from standard curves constructed using fluoride standards (10⁻⁷ to 10⁻³ M NaF) prepared from a commercially available standard solution (10⁻¹ M NaF).

Comparison of the defluorination of D₂-sevoflurane and sevoflurane in microsomes incubated with an excess of either anesthetic (1 µl anesthetic per incubation) shows that D₂-sevoflurane is defluorinated much slower than sevoflurane in all microsomal preparations (table 1).

**Table 1**

| **COMPARATIVE DEFLUORINATION OF SEVOFLURANE AND D**_{**2**}**-SEVOFLURANE BY RAT LIVER MICROSOMES*** | | |
|---|---|---|
| nmol F⁻ /mg protein/30 min ± S.E. | | |
| Animal Treatment | Sevoflurane | D₂-Sevoflurane |
| None | 1.94 ± 0.31 | 0.62 ± 0.60 **(68)*** |
| Isoniazid | 7.46 ± 0.83 | 1.55 ± 0.39 **(79)** |
| Phenobarbital | 1.18 ± 0.06 | 0.18 ± 0.03 **(84)** |

| | | |
|---|---|---|
| * Numbers in parentheses represent percent decline from sevoflurane values following correction for background (0.43). Values represent the mean and standard errors of triplicate determinations. | | |

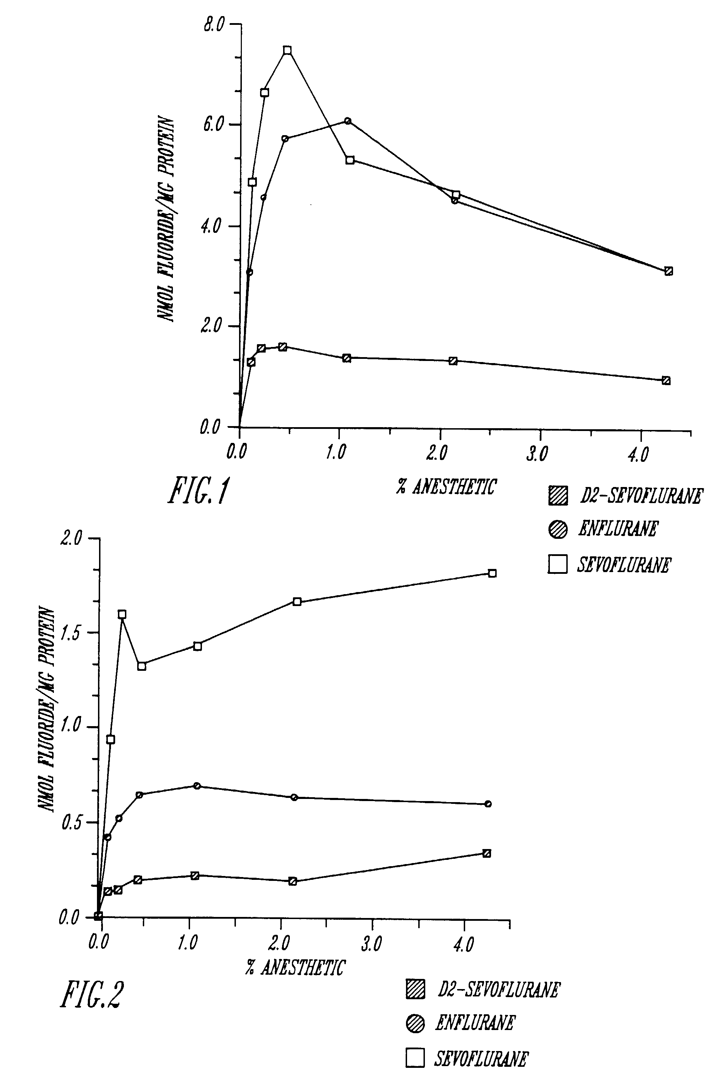

The degrees of inhibited metabolism are 68, 79 and 84% in microsomes from untreated, isoniazid and phenobarbital treated rats, respectively. The concentration-dependent defluorination of D₂-sevoflurane, sevoflurane and enflurane, in microsomes from phenobarbital and isoniazid treated rats show that over a wide range of anesthetic concentrations D₂-sevoflurane is defluorinated substantially slower than sevoflurane (70-86% less) or enflurane (figures 1 and 2). In microsomes from isoniazid treated rats in which the metabolism of all anesthetics is the greatest due to the induction of P450 IIE1, there was an anesthetic concentration-dependent inhibition of metabolism by sevoflurane and enflurane, but not D₂-sevoflurane (figure 1). These data suggests a substrate inhibition phenomenon. In microsomes from rats treated with phenobarbital this did not occur (figure 2).

### Example 5

### IN VIVO METABOLISM OF D₂-SEVOFLURANE

Untreated rats or rats treated with isoniazid or phenobarbital were exposed to D₂-sevoflurane, sevoflurane, or enflurane to determine the relative rates of fluoride production in vivo.

The animals were exposed in a 3.8 L plastic exposure chamber with an atmosphere of 100% oxygen. Male Sprague-Dawley rats (200-220g, 4 per group) were placed in the chamber and the chamber was flushed with oxygen and sealed. Anesthetic was introduced into the chamber in liquid form via an injection port. Quantities were introduced to give initial concentrations of 3% anesthetic (enflurane, 464 µl; sevoflurane and D₂-sevoflurane, 524 µl). The rats became anesthetized within 4-6 minutes after introduction of each anesthetic. Oxygen and carbon dioxide were monitored periodically during the exposure period with an Ohmeda 6000 multi-gas monitor.

Following a 30 minute exposure period, the chamber was flushed with 100% oxygen for 5 minutes and the animals rapidly awakened. The rats were immediately removed and injected i.p. with 80 mg/kg secobarbital. While anesthetized 3 to 4 ml of blood were withdrawn by cardiac puncture within 15 minutes of termination of anesthetic exposure (within 10 minutes of removal from the chamber). Plasma was prepared and fluoride analyzed as described above.

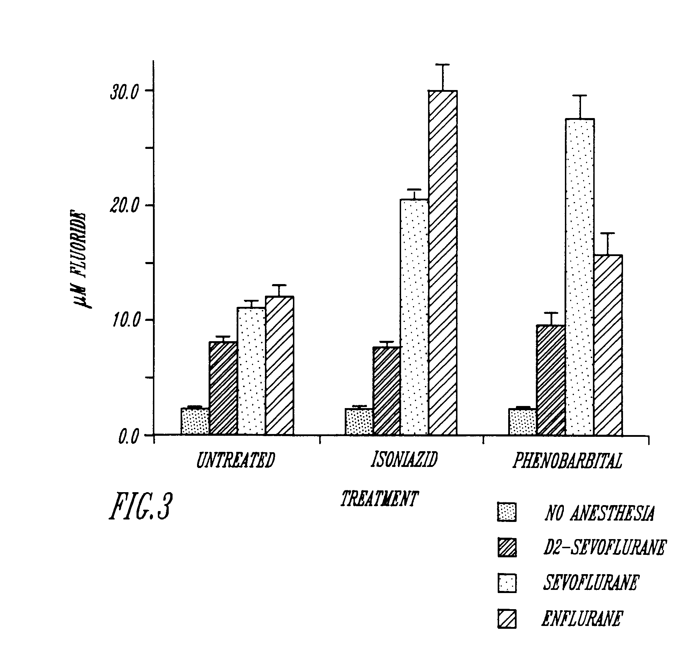

Exposure to D₂-sevoflurane resulted in lower plasma fluoride than exposure to either enflurane or sevoflurane (figure 3). As compared to the liberation of fluoride from sevoflurane, D₂-sevoflurane liberated 61% less in isoniazid treated rats, 66% less in phenobarbital treated animals, and 34% less in untreated rats. D₂-sevoflurane also liberated less fluoride than enflurane *in vivo.* In untreated, and in isoniazid and phenobarbital treated rats, the plasma from D₂-sevoflurane exposed rats contained 40, 80, and 45%, respectively, less fluoride than enflurane anesthetized animals.

### Experiment 6

An anesthesia circuit containing fresh soda lime (1.5 kg) was set up with an anesthesia machine (Foregger F 500). Sevoflurane at an initial concentration of 2.7% (a relatively high anesthetic concentration) was added to the circuit and 100% oxygen was used as the makeup gas. The gases were re-circulated at a total circuit flow rate of 1.5 liters per minute (a relatively low clinical total flow rate) in a closed system with a respirator. Carbon dioxide was added to the circuit at a constant rate of 200 ml per minute. This set up the conditions for "worst case" breakdown of sevoflurane by soda lime.

Experiments were conducted at exothermic temperature versus with the soda lime canister immersed in an ice bath. When the experiments were conducted at exothermic temperatures, the temperature in the core of the soda lime stabilized at between 44°C and 47°C after about one hour. Degradation products from the breakdown of sevoflurane were detected at about 1.5 hours after the circuit was started. Products were continually detected for the remainder of the experiments (up to eight hours) by gas chromatography. However, when the canister was immersed in the ice bath, the core temperature of the soda lime canister equilibrated at between 22°C and 27°C when stabilized. It never increased above this range. Using the same chromatography analysis, no degradation products were detected in this system, which was also run for up to eight hours. Carbon dioxide levels on the respiratory side of the circuit were completely scrubbed and were no different between soda lime in ice or at ambient temperature.

Incubations of sevoflurane with soda lime in static systems also showed this same effect. At 22°C some degradation products including "Compound A" were detected; however, at 45°C substantial degradation products, including "Compound A", were produced. Similar experiments were run with deuterated sevoflurane and results were essentially the same.

From these experiments, it was concluded that maintaining the soda lime temperature in the range of 4°C to 27°C reduced the production of volatile degradation by-products of sevoflurane or deuterated sevoflurane to virtually insignificant levels.

## Claims

1. A deuterated sevoflurane having the following formula:- 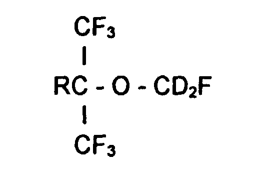 wherein R is hydrogen or deuterium.

2. Fluorodideuteromethyl-1, 1,1,3,3,3 -hexafluoro-2-propyl ether.

3. A method of synthesising fluorodideuteromethyl-1 ,1,1,3,3,3-hexafluoro-2-propyl ether, the method comprising mixing dimethyl-D₆-sulfate with 1,1,1,3,3,3-hexafluoroisopropanol and reacting said mixture with bromine trifluoride to produce fluorodideuteromethyl-1, 1,1,3,3,3-hexafluoro-2-propyl ether.

4. A method of synthesising fluorodideuteromethyl-1 1,1,3,3,3-hexafluoro-2-deutero-2-propyl ether, the method comprising reacting fluorodideuteromethyl-1 1,1,3,3, 3-hexafluoro-2-propyl ether with sodium deuteroxide in D₂O to produce fluorodideuteromethyl-1, 1,1,3,3, 3-hexafluoro-2-deutero-2-propyl ether.

5. An inhalational anaesthetic composition for inducing anaesthesia in animals, the composition comprising deuterated sevoflurane and an anaesthetic carrier, said deuterated sevoflurane being a deuterated sevoflurane as claimed in Claim 1.

6. A composition as claimed in claim 5 wherein said anaesthetic carrier is oxygen.

7. Deuterated sevoflurane for use as an anaesthetic, said deuterated sevoflurane being a deuterated sevoflurane as claimed in Claim 1.

8. Use of deuterated sevoflurane for the manufacture of a medicament for use in anaesthesia, said deuterated sevoflurane being a deuterated sevoflurane as claimed in Claim 1.

9. Use of deuterated sevoflurane for the manufacture of an anaesthetic agent for use in the medical or surgical treatment of an animal in need thereof by the practice of therapy, prophylaxis or surgery exercised while the animal subject is anaesthetised by said anaesthetic agent, said deuterated sevoflurane being a deuterated sevoflurane as claimed in Claim 1.

## Patentansprüche

1. Ein deuteriertes Sevofluran mit der nachstehenden Formel: 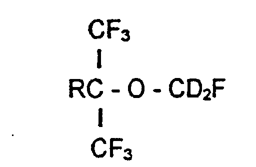 in der R Wasserstoff oder Deuterium ist.

2. Fluordideuteromethyl-1,1,1,3,3,3 -hexafluor-2-propylether.

3. Verfahren zur Herstellung von Fluordideuteromethyl-1,1,1,3,3,3-hexafluor-2-propyl-ether, wobei das Verfahren umfaßt, Vermischen von Dimethyl-D₆-sulfat mit 1,1,1,3,3,3-Hexafluorisopropanol und Umsetzen dieses Gemisches mit Bromtrifluorid, um Fluordideu-teromethyl-1,1,1,3,3 ,3-hexafluor-2-propylether herzustellen.

4. Verfahren zur Herstellung von Fluordideuteromethyl-1,1,1,3,3,3-hexafluor-2-deutero-2-propylether, wobei das Verfahren umfaßt, Umsetzen von Fluordideuteromethyl-1,1,1,3,3,3-hexafluor-2-propylether mit Natriumdeuteroxid in D₂O, um Fluordideuteromethyl-1,1,1,3,3,3-hexafluor-2-deutero-2-propylether herzustellen.

5. Eine Inhalationsnarkosemittel-Zusammensetzung zur Herbeiführung von Narkose bei Tieren, wobei die Zusammensetzung deuteriertes Sevofluran und einen Narkosemittel-Trägerstoff umfaßt, wobei das deuterierte Sevofluran ein deuteriertes Sevofluran gemäß Anspruch 1 ist.

6. Zusammensetzung nach Anspruch 5, wobei der Narkosemittel-Trägerstoff Sauerstoff ist.

7. Deuteriertes Sevofluran zur Verwendung als Narkosemittel, wobei das deuterierte Sevofluran ein deuteriertes Sevofluran gemäß Anspruch 1 ist.

8. Verwendung von deuteriertem Sevofluran zur Herstellung eines Medikaments zur Verwendung bei der Narkose, wobei das deuterierte Sevofluran ein deuteriertes Sevofluran gemäß Anspruch 1 ist.

9. Verwendung von deuteriertem Sevofluran zur Herstellung eines Narkosemittels zur Verwendung bei der medizinischen oder chirurgischen Behandlung eines Tieres, das diese benötigt, durch die Anwendung von Therapie, Prophylaxe oder chirurgische Behandlung, die ausgeführt wird, während das Tier durch dieses Narkosemittel narkotisiert ist, wobei das deuterierte Sevofluran ein deuteriertes Sevofluran gemäß Anspruch 1 ist.

## Revendications

1. Sévoflurane deutéré ayant la formule suivante : 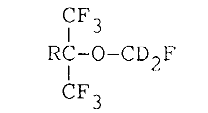 où R est l'hydrogène ou le deutérium.

2. Éther de fluorodideutérométhyle et de 1,1,1,3,3,3hexafluoro-2-propyle.

3. Procédé de synthèse de l'éther de fluorodideutérométhyle et de 1,1,1,3,3, 3-hexafluoro-2-propyle, le procédé consistant à mélanger du sulfate de diméthyle-D₆ avec du 1,1,1,3,3,3-hexafluoro-isopropanol et à faire réagir ledit mélange avec du trifluorure de brome pour produire l'éther de fluorodideutérométhyle et de 1,1,1,3,3,3-hexa-fluoro-2-propyle.

4. Procédé de synthèse de l'éther de fluorodideutérométhyle et de 1,1,1,3,3,3-hexafluoro-2-deutéro-2-propyle, le procédé consistant à faire réagir l'éther de fluorodideutérométhyle et de 1,1,1,3,3,3 -hexafluoro-2-propyle avec du deutéroxyde de sodium dans D₂O pour produire l'éther de fluorodideutérométhyle et de 1,1,1,3,3,3-hexafluoro-2-deutéro-2-propyle.

5. Composition anesthésique à inhaler destinée à provoquer une anesthésie chez des animaux, la composition comprenant du sévoflurane deutéré et un véhicule d'anesthésique, ledit sévoflurane deutéré étant un sévoflurane deùtéré tel que revendiqué dans la revendication 1.

6. Composition telle que revendiquée dans la revendication 5, dans laquelle ledit véhicule d'anesthésique est l'oxygène.

7. Sévoflurane deutéré à utiliser comme anesthésique, ledit sévoflurane deutéré étant un sévoflurane deutéré tel que revendiqué dans la revendication 1.

8. Utilisation de sévoflurane deutéré pour la fabrication d'un médicament à utiliser en anesthésie, ledit sévoflurane deutéré étant un sévoflurane deutéré tel que revendiqué dans la revendication 1.

9. Utilisation de sévoflurane deutéré pour la fabrication d'un agent anesthésique à utiliser dans le traitement médical ou chirurgical d'un animal qui en a besoin en raison de la mise en oeuvre d'une thérapie, d'une prophylaxie ou d'une chirurgie exercée tandis que le sujet animal est anesthésié par ledit agent anesthésique, ledit sévoflurane deutéré étant un sévoflurane deutéré tel que revendiqué dans la revendication 1.
